# EUROPEAN PATENT SPECIFICATION

(11) **EP 3 313 489 B1**
(45) Date of publication and mention of the grant of the patent: **09.02.2022**
(21) Application number: 16736338.1
(22) Date of filing: 23.06.2016
(51) Int. Cl.: A61M 16/06

(54) **POSITIVE PRESSURE MASK AND RELATED ADAPTERS AND APPLICANCES**
POSITIVDRUCKMASKE UND ZUGEHÖRIGE ADAPTER UND GERÄTE
MASQUE À PRESSION POSITIVE ET ADAPTATEURS ET DISPOSITIVS

(30) Priority: 23.06.2015 US 201562183733 P; 08.11.2015 US 201562252577 P
(43) Date of publication of application: 02.05.2018
(73) Proprietor: ReddyPort, Inc., Salt Lake City, UT 84101 (US)
(72) Inventor: REDDY, Chakravarthy, B., Salt Lake City, UT 84124 (US); ORR, Joseph, Jeremy Ranch, UT 84098 (US); NIEMAN, Timothy, R., North Salt Lake, UT 84054 (US); SPENDLOVE, Jared, South Weber, UT 84405 (US); ROSENHAN, Branden, R., Salt Lake City, UT 84106 (US); HANSEN, Andrew, S., Bountiful, UT 84010 (US)
(74) Representative: Sach, Greg Robert
(86) International application number: PCT/US2016/039117
(87) International publication number: WO 2016/210192

(56) References cited:
- WO-A1-2015/054747
- US-A1- 2003 047 189
- US-A1- 2007 244 426
- US-A1- 2012 285 465
- US-B1- 8 365 734

## Description

### CROSS-REFERENCE TO RELATED APPLICATIONS

This Application claims the benefit of U.S. Provisional Patent Applications Nos. 62/183,733, filed June 23, 2015 and 62/252,557, filed November 8, 2015, and titled Oral Access Airway Devices.

### BACKGROUND

### 1. Field of the Invention

The present invention relates to devices for providing oral access with a non-invasive positive pressure mask.

### 2. Related Technology

Non-invasive positive pressure masks are currently used in the medical field for patients with poor oxygen saturation, sleep apnea, and other related respiratory problems. The mask includes a peripheral flexible membrane that contacts the face of the patient and creates a seal with the face using the positive pressure. An example of a positive pressure ventilation mask is disclosed in US Patent 6,513,526 to Kwok. These types of masks, when used with a ventilator, provide positive pressure airflow without the need to intubate the patient and can prevent the need for tracheal intubation or allow earlier extubation.

Positive pressure masks require an effective seal around the facial area and can be somewhat difficult or time consuming for the user or patient to properly place. Once in place positive pressure mask provide the amount of forced air necessary to maintain adequate inhalation and exhalation. In a matter of hours or days, the mask can cause discomfort to the patient from dry mouth or nose, nasal congestion, rhinitis or runny nose, facial irritations, bloody noses, dry mucosal tissue, dry lips, increased risk of respiratory infection, or other difficulties managing oral or nasal airway.

Positive pressure masks are also used to treat sleep apnea. While these patients are typically not critically ill, they suffer from the inconvenience of dryness of the airway and the inability to access their oral airway without taking the mask off.

### SUMMARY

The present invention relates to positive air pressure masks and elbows for providing oral or nasal access to a patient in need of non-invasive positive air pressure ventilation. Described are devices and methods that utilize a valve in the mask to provide access to the oral or nasal cavity. The valve is preferably self-sealing under pressure and self-reverting (i.e., non-inverting). Described is oral care on a patient while maintaining sufficient pressure to provide clinically relevant breathing assistance and/or to maintain an open airway in a patient.

Described is a method for providing oral care through a positive pressure ventilation mask. The method includes providing a positive pressure ventilation mask including a mask body defining a cavity and having a peripheral seal configured to contact a face of a wearer. The mask includes an access port providing external access to the cavity and an inlet for receiving pressurized air. The method also includes providing an oral care appliance with an adapter configured to seal with the port. The method includes pressurizing the mask to a pressure of at least 200 Pa using a ventilator system and introducing the oral care appliance into the cavity through the port and maintaining a pressure of at least 200 Pa while performing an oral care procedure.

An embodiment of the invention relates to a ventilation mask for providing oral or nasal care. The mask includes aa mask body defining a cavity and having a peripheral seal configured to engage a wearer's face under pressure. A port in the mask body is configured to receive pressurized air; The mask includes a self-sealing access valve configured to provide direct external access to the cavity, wherein the access valve has an open diameter of at least 10 mm and self-seals under a pressure of 500-2500 Pa or 5-25 cm H₂O.

Described is also an oral care module. The module includes an oral care appliance and an appliance adapter for inserting the oral care appliance through a valve in a positive pressure mask. The appliance adapter includes a body having an insertion portion configured for insertion through a valve of a ventilation mask, a sealing portion configured to engage the valve and create a seal therebetween. An external portion (e.g., a handle) is configured for manually manipulating the appliance adapter. A receptacle is formed in the body and sized and configured to receive a working portion of the oral appliance. The receptacle has a first opening in the insertion portion of the body and a second opening opposite the first opening, wherein the first opening is sized and configured to pass the working portion of the appliance therethrough and the second opening is sized and configured to provide a seal around a shaft of the appliance. US2003/047189 describes a face mask with an airtight seal for providing positive pressure ventilation using medical devices passing through a second medical port.

### DESCRIPTION OF DRAWINGS

Figure 1 illustrates a full face positive pressure mask, including a valve adapter, secured to the face of a wearer;
Figure 2 shows the access adapter of the face mask of Figure 1;
Figure 3 is an exploded view of the access adapter of Figure 1;
Figure 4 is a cutaway view of the access adapter of Figure 1;
Figure 5 is a perspective view of the bottom side of an access valve of the access adapter of Figure 1;
Figure 6 illustrates an appliance module including an appliance adapter and a suction brush;
Figure 7 is a cutaway view of the module of Figure 6;
Figure 8 shows an exploded view of the module of Figure 6;
Figure 9 shows an assembly of the module of Figure 6 and the access adapter of Figure 2.

### DETAILED DESCRIPTION

### Positive Pressure Ventilation Mask With Self-Sealing Valve

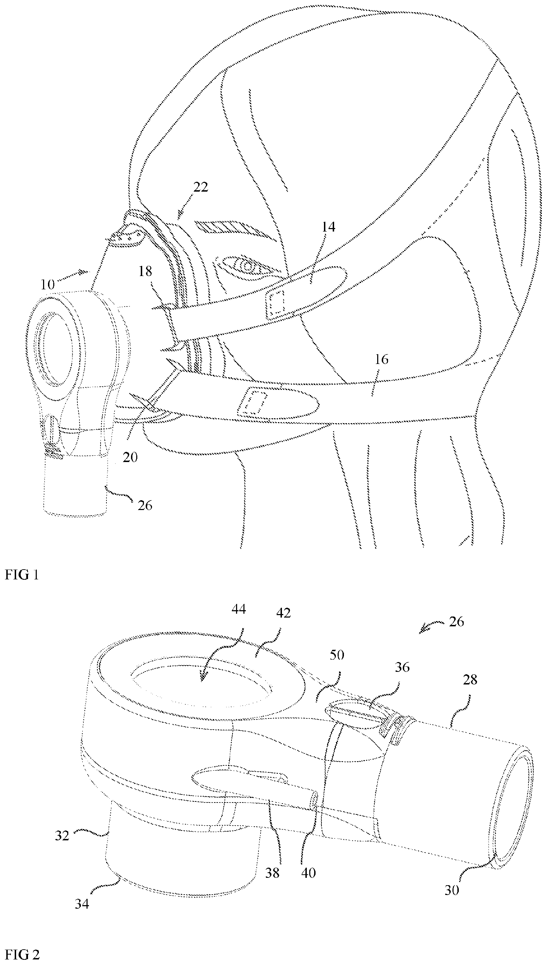

The invention includes devices that utilize a valve in the mask to provide access to the oral or nasal cavity. The valve is preferably self-sealing under pressure and/or self-reverting (i.e., non-inverting). The valve can be positioned in the mask body or in a valve adapter. In one embodiment the valve adapter may be a valve integrated into an air supply connector such as the mask elbow (Figure 2).

The positive pressure masks of the present invention include a mask body defining a cavity. Attached to the body is a peripheral flexible membrane configured to engage a wearer's face and form a seal. Masks having membranes suitable for sealing around the mouth and nose of a patient using positive pressure are described in US. patents 9,295,799 to McAuley; 6,513,526 to Kwok, and D464,728 to Paul. The masks are configured to be fluidly coupled to a ventilator through an air supply connector such as an elbow. The ventilator used with the masks of the invention are preferably bi-level pressure ventilators (or alternatively continuous pressure ventilators). Bi-level ventilation is typically important for critical care patients.

Figure 1 illustrates a positive pressure mask 10 that includes a mask body 12. Mask body 12 is secured to the head of the patient using upper strap 14 and lower strap 16. Straps 14 and 16 connect to eyelets 18 and 20, respectively, on mask body. Straps 14 and 16 connect to eyelets on corresponding locations (not shown) on an opposite side of body 12. The straps secure the mask to the head and avoids movement of the mask relative to the head that could cause air leaks that diminish the positive air treatment. At the periphery of the mask body 12, mask 10 includes a flexible membrane 22 (i.e., cushion) that has a flap that can form a seal with the face of the patient when positive pressure is delivered from a ventilation system 24 through valve adapter 26 and into an opening 28 in mask body 12. The cavity of mask body 12 forms a positive air pressure chamber between it and the face of the patient.

Figures 2-4 show the valve adapter in more detail. Valve adapter 26 includes a adapter body 50 formed from upper housing 52 and lower housing 54. An access valve 42 is secured to upper housing 52 using locking ring locking ring 46. Adapter 26 also includes an anti-asphyxiation valve that uses a flap 48 to open and close aperture 36. For purposes of this invention, unless otherwise stated or implied, the term "valve" by itself refers to the "access valve".

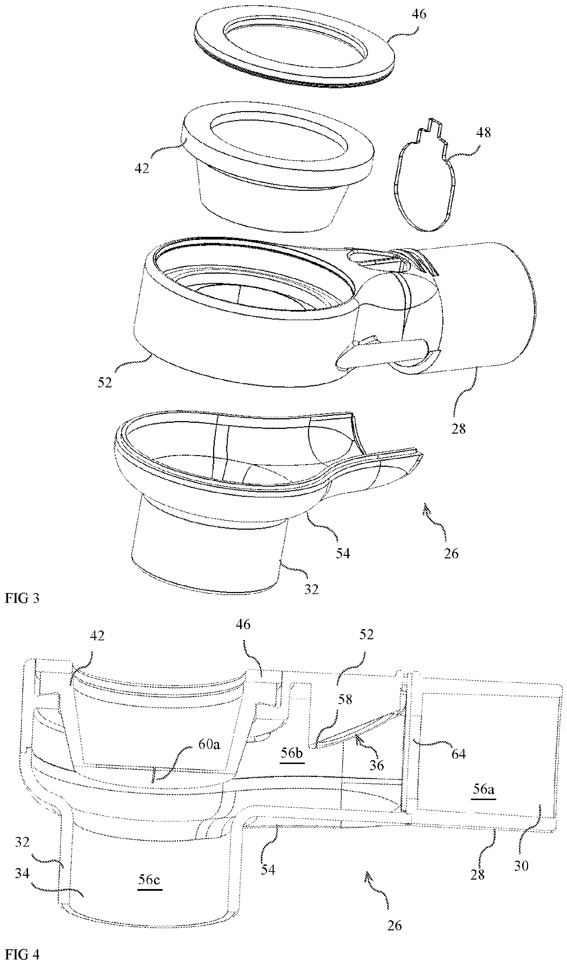

Valve adapter 26 is an elbow type air supply connector that includes an airdelivery conduit. The air supply conduit extends between inlet 30 and outlet 34 and includes internal regions 56a, 56b, and 56c (Figure 3). Valve 42 is in fluid communication with the air delivery conduit in region 56c. Valve 42 provides access to a wearer's mouth and nose through external opening 44 (i.e., port 44) and region 56c of the conduit.

The air supply conduit provided by valve adapter 26 is configured to deliver pressurized air from a source of positive air pressure (e.g., ventilator hose) to the cavity of the ventilation mask 10. Air pressure in inlet 30 forces flap valve to open to provide fluid communication between regions 56a and 56b. The air flow between region 56a and 56b forces flap 48 upward to close off aperture 36 by seating against seat 58. If air flow stops between regions 56a and 56b, flap 48 drops down to opening 64 to prevent air from flowing backwards through inlet 30 (i.e., from region 56a to 56b). Flap 48 prevents asphyxiation by allowing air to be breathed from the ambient (through aperture 36) if the supply of air from the ventilator is interrupted.

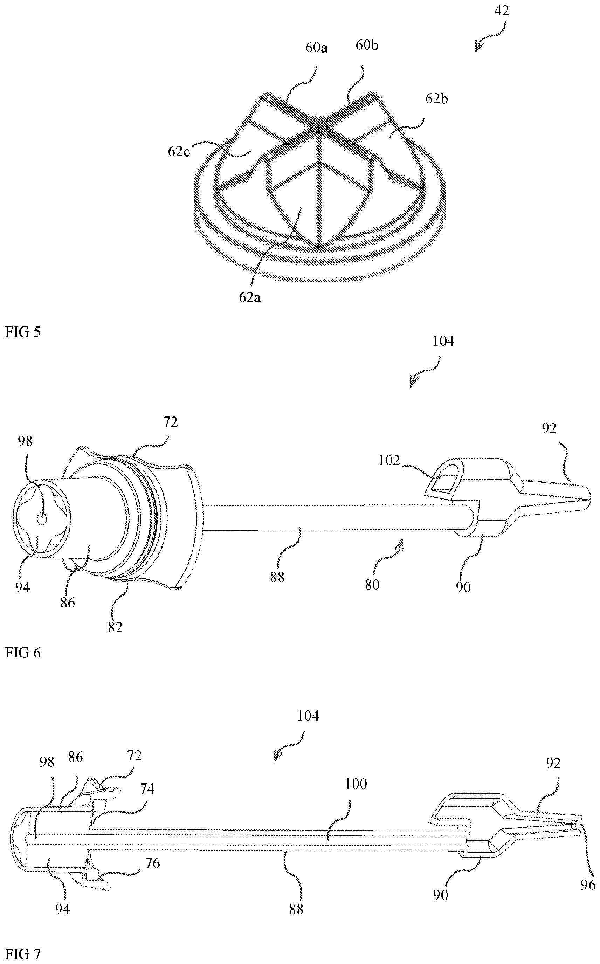

Valve adapter 26 includes a first press-fit connector 28 that serves to fluidly connect a positive pressure air supply hose (not shown) to inlet 30 of valve adapter 26. A second press-fit connector 32 serves to fluidly connect the outlet 34 of valve adapter 26 to an inlet in mask body 12. The press-fit connection may be configured to be sufficiently tight that when an appliance is positioned in the adapter (Figure 7) and pulled out of valve 42, the press fit maintains the connection of the air supply connector to the mask.

Adapter 26 preferably swivels relative to mask body 26 such that a hose connected to adapter 26 can be redirected without torqueing the mask. Any swivel mechanism can be used. The swivel mechanism may be incorporated into a mask body, a valve adapter, or the connection therebetween.

Connections other than press-fit may be used to connect a valve adapter to a mask or ventilation system, including non-removable connections and quick release connections.

Valve adapter 26 may also include a pressure port 40 on stem 38. Pressure port 40 includes a small opening in fluid communication with region 56b that is used to monitor pressure changes in valve adapter 26. Changes in pressure can be used to detect when the wearer of the mask is inhaling or exhaling. Bi-level pressure ventilators can use the pressure port 40 to provide lower pressure during exhalation and increased pressure during inhalation. Pressure port 40 is not required to be associated with adapter 26, but rather can be placed in mask body 12 or both.

The ventilation masks of the present invention (including adapter 26) use a self-sealing access valve. The self-sealing access valve can be placed anywhere on the mask that allows direct external access to the mouth or nose of the patient (i.e., access to the mouth or nose through the mask). The access valve has an open diameter sufficient to perform oral care or insert appliance that would cause excessive leaking if inserted through a valve that is not self-sealing. The diameter of the opening in the self-sealing valve (in the open position) can be at least 10, 15, or 20 mm and/or less than 30, 25, or 20 mm and/or within a range of the foregoing. These diameters of opening can be achieved with a valve that will be self-sealing under pressures of 5-25 cm H₂O (approximately 500-2500 Pa). In some embodiments the opening in the access valve is provided by one or more slits. The length of the slit may provide the maximum open width. In some embodiments the valve includes a plurality of slits. In some embodiments the valve can include 2 slits and the slits may be a cross-slit.

To facilitate self-sealing, the access valve may have inward sloping walls or concavity. The valve may be a duck bill valve or a dome shaped valve. The valve may include fenestrations that form leaflets. The valve may include a plurality of leaflets. The leaflets may be configured to be pushed open by an appliance or appliance adapter from the outside and pushed together by pressure from the inside. A duck bill valve may have a plurality of leaflets. Figure 5 shows a bottom perspective view of valve 42 in greater detail to illustrate a cross slit and a plurality of leaflets. Valve 42 includes two slits 60a and 60b, and four leaflets 62a, 62b, and 62c (the fourth leaflet is not visible in Figure 5) (collectively referred to as slits 60 and leaflets 62, respectively).

Where a dome valve is used, the dome may have a tapered thickness that is thin at a center opening and tapers to a greater thickness towards the edges. The taper may include a change of thickness greater than 1.2, 1.5, or 2 times the thickness at a lateral edge of a fenestration as compared to a center edge of a fenestration. The taper may allow the valve to open more easily at the center.

In a preferred embodiment, the valve reverts itself if it becomes inverted (i.e., self-reverting). For purposes of this invention, a self-reverting valve has a material and configuration that causes the valve to return to its self-sealing position when inverted. Thus, if an instrument is pulled out of the valve and a leaflet or other component is inverted, the self-reverting valve returns to its self-sealing position once the force is removed. Although not required, the valve may be concave and/or made of a silicone material (or similar material) to facilitate self-reverting. In one embodiment, the valve includes a layer of material at its center that is less than 5, 4, 3, or 2 mm thick.

The access valve and/or combination of one or more of the access valve, anti-asphyxiation valve, and mask may be configured to have a leak rate less than 70, 50, 40, 30, or 25 liters per minute ("1pm") and/or greater than 2, 5, 7, or 10 1pm and/or within a range of the foregoing when the mask is under an air pressure of at least 5, 10, 15, cm H₂O and/or less than 25, 20, or 15 cm H₂O or within a range of the foregoing. For purposes of this invention, the leak rate is measure

In some embodiments, the valve may include a biocompatible lubricant to facilitate insertion of appliances or appliance adapter through the valve. The access valve and/or lubricant may also include an anti-microbial agent (e.g., chlorhexidine).

### Appliances, Adapters, Modules, and Kits For Performing Oral/Nasal Procedure Under Positive Pressure

Described are appliances (e.g., scrub brush, suction device) that have an adapter configured to mate with a port (e.g., access valve 42) on a positive pressure mask and provide a seal sufficient to maintain clinically relevant positive pressure while the appliance is being used. The appliance adapter reduces the amount of leaking caused by inserting the appliance through the mask port as compared to inserting the appliance through the port without the appliance adapter. The sealing structure may be a membrane, gasket, or appropriately sized aperture configured to engage a structure of the appliance (e.g., a shaft or tubing).

The port preferably includes a self-sealing access valve (e.g., the self-sealing and self-reverting access valves as described herein) and is configured to engage the appliance adapter to seal off the access valve. The use of an adapter that seals off a self-sealing valve allows the use of different adapters tailored to particular appliance with the same type of valve in the mask. The same self-sealing access valve in a mask can be useful for numerous different procedures and appliances. This flexibility can be important because the particular procedures that may need to be performed on a patient are not known when the mask is placed and emergency situation may call for different types of procedures where removing the mask is clinically undesirable.

In addition or alternatively, the appliance adapter may have a flexible membrane sized and configured to seal around a particular appliance. The flexible membrane around the appliance allows the appliance to be manipulated through a port by flexing the membrane. The ability to flex the membrane is important for being able to carry out procedures that require extensive manipulation (e.g., many oral care procedures). The membrane can be a separate piece connected to the body or the membrane may be an integral component of the (i.e., the membrane is a single injection molded piece with the appliance, but is made thin so as to provide flexibility as compared to the rest of the body).

When coupling the appliance adapter to a mask with a self-sealing valve, the appliance adapter may have a receptacle for housing a compressible material such as a sponge. The receptacle may provide access through the self-sealing valve without compressing the material.

The appliance adapter allows an appliance to pass through the valve adaptor protected from the pressure of the valve. For instance, an appliance may include a sponge that is protected from being squeezed in the valve. The appliance adapter can allow for appliances to be inserted and withdrawn while still having fluids on them.

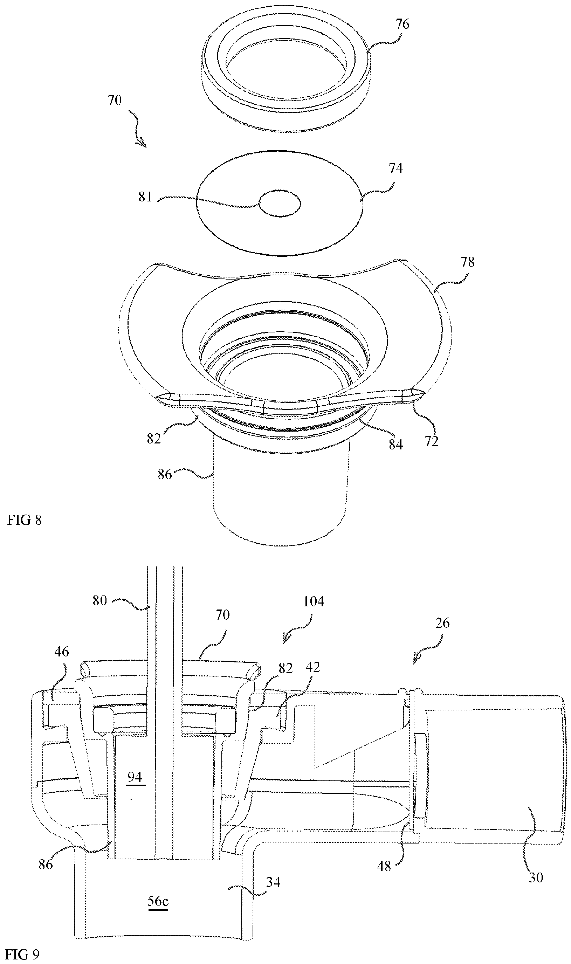

Figures 6-8 illustrate an example of an appliance adapter 70 assembled with an oral care suction brush 80 to form an appliance module 104. Appliance adapter 70 includes an adapter body 72. Adapter body 72 receives a membrane 74 that is retained by a retaining ring 76. Retaining ring 76 connects to body 72 and positions membrane 74 therebetween. Appliance membrane 74 include an aperture 81 for receiving a shaft of an appliance such as suction brush 80. Appliance body 72 has a receptacle 86 configured to pass through access valve 42 (Figure 9). Receptacle 86 provides an opening to the inside of a mask. Receptacle 86 is sealed by membrane 120 on a first end and open on an opposite end. Receptacle 86 is configured to store a working piece of an appliance (e.g., a sponge on a shaft.).

Adapter body 72 also has a sealing structure 82 (i.e., rim portion) configured to provide a seal when module 104 is inserted into valve adapter 26. Ring portion 78 provide a handle for gripping appliance adapter 70.

Sealing structure 82 may have any configuration that will removably engage and form a seal with the valve. In one embodiment sealing structure 82 may include an annular bump 84 to provide a seal against valve 42 with minimal friction upon removing adapter 70. Annular bump 84 may engage a wall of valve 42 or another feature of valve adapter 26 or valve 42 (e.g., an annular bump on valve 42 or ring 46). In one embodiment appliance adapter engages a vertical portion of valve 42 with an angle of less than 10, 5, 2, 1 degrees. The vertical portion of valve 42 where appliance adapter 70 engages valve 42 may be at least 3, 5, 7, or 10 mm.

Suction brush 80 includes a shaft 88, a handle portion 90, and a brush portion 94. Handle portion includes a connector for attaching a source of suction to an outlet 96 (i.e., air is drawn out o outlet 96 from negative pressure).

Shaft 88 has a suction channel 100 that extends from suction aperture 98 to outlet 92. The fluid pathway between suction aperture 98 and outlet 92 is in fluid communication with suction by-pass port 102. Suction through aperture 98 may be selected by closing port 102 (e.g., using a finger to occlude the opening). By closing port 102, suction is directed to suction aperture 98. The suction mechanism of suction brush 80 may vary. For instance, handle 90 may include an open and close switch rather than a by-pass. In addition, shaft 88 may include additional or different openings for suctioning from one or more different locations within or near the brush area.

The appliance adapter may be an endoscope adapter that includes a sealing structure (e.g., gasket, membrane, or appropriately sized aperture) configured to form a seal with an endoscope. The endoscope may be used to perform an esophagogastroduodenoscopy (EGD) procedure while maintaining pressure in a mask. The opening in the appliance adapter for accommodating the endoscope may be at least 8, 10, or 12 mm and/or less than 15, 13, or 11 mm and/or within a range of any of the foregoing maximum and minimum dimensions.

Another type of adapter that can be used in the present invention is a series of leak adapters configured to have a particular leak rate when placed in a port (e.g., a self-sealing valve). The selected leak can be caused using a particularly shaped structure of the adapter in connection with the access valve, air supply connector, or mask body. The adapter may have holes, slits, or other structures configured to leak at a particular rate when connected to a particular mask.

Described is also a nebulizer adapter. The nebulizer adapter has a connector for coupling to a source of nebulized case and a second connector for coupling to the positive air pressure mask. For example, appliance adapter 70 may be used and then modified to couple to a nebulizer. The coupling to the nebulizer may be any technique known in the art.

Figure 9 illustrates the operation of a module 104 and valve adapter 26. Appliance adapter 70 is inserted into valve 42 of adapter 26 and sealing structure 82 of adapter 70 forms a seal with valve 42. Structure 82 may include an annular bump to create a desired friction for retaining appliance adapter 70 within valve adapter 26. alternatively the inner surface of valve 42 may have an annular bump (not shown) that engages a bump on appliance adapter to provide a seal.

Receptacle 86 forces slits 60 open and leaflets 62 (shown in Figure 5) flex to create an opening in valve 42 that accommodates the receptacle, and thus sponge 94 housed therein. Receptacle 86 forms the insertion portion of appliance adapter 70.

The receptacle 86 has an open end opposite membrane 74. The open end is configured to allow the working surface (e.g., sponge 94) of the appliance to freely pass therethrough. The open end may have a diameter in a range from 5, 10, 15, or 20 cm and/or less than 40, 35, 30, 25, 20, or 15 cm and/or within a range of any of the foregoing.

The appliance may need to have a shaft that is longer than a corresponding traditional appliance to accommodate for the additional thickness of the valve and/or valve adapter.

In use valve adapter 26 is in fluid communication with mask body 12 at region 56c and in fluid communication with a positive air supply from a ventilator at inlet 30 and flap valve 48 would move upward to open the conduit between inlet 30 and outlet 34.

Scrub brush 80 can be advanced into the mouth of a patient through region 56c of valve adapter 26 while membrane 74 creates a seal around shaft 88 of suction brush 80. Because receptacle 86 does not fully occlude the space in region 56c, airflow and thus positive pressure can continue to pressurize the chamber between the mask and the face of the patient. Fluids can be delivered to the mouth in sponge 94 and suction can be used to remove the fluids from the mouth through suction brush 80. Because membrane 74 is flexible, suction brush 80 can be manipulated in different directions, while still keeping a relatively good seal.

To remove sponge 94, scrub brush 80 is retracted until sponge 94 is again housed within receptacle 86. Valve adapter 70 is removed from valve 42 and valve 42 self-seals from the positive air pressure within region 56c pushing against leaflets 62 of valve 42, thereby causing slits 60 to close. If the user does not fully retract sponge 94 into receptacle 86 and the sponge catches on leaflets 62 and caused the valve to invert, the configuration of the leaflets 62 and the resilient material cause it to revert itself (i.e., self-reverting) under pressures described herein for positive pressure ventilation.

The foregoing masks, valves, modules, and/or appliances can be used to provide an oral or nasal care system for cleaning the oral or nasal passageways of a patient on a positive air pressure ventilation mask.

While the appliance adapters of the present invention have been illustrated with a self-sealing, self-reverting valve, unless otherwise stated, embodiments of the invention also include performing oral access procedures under pressure with appliances and appliance adapters that are configured to attach to a port with a mere aperture and a removable cap or the like. (see e.g., aperture 22 of US Patent application publication 2010/0116276 to Bayasi).

The appliances described herein may be packaged with the appliance adapter and/or be assembled and/or packaged together. In the case where the seal between the appliance adapter and the appliance is specific to the appliance, the appliance adapters typically needs to be preassembled with the appliance. For example, a suction brush may have a handle on one end and a sponge brush on the other end of a tube and the seal needs to engage the tube. In some cases pre-assembly of the appliance adapter and corresponding appliance is necessary to avoid the need to find the correct adapter when performing a particular procedure. For instance, when performing an endoscopic procedure an appliance used to introduce anesthetic is best assembled with the adapter to avoid the situation where a practitioner needs to use the appliance to introduce anesthetic and does not have an appliance adapter readily available. Nevertheless, when the appliance is expensive and non-disposable (e.g., an endoscope), the integration of the appliance adapter may not be practical, despite the advantage of doing so.

### Methods For Treating A Patient While Maintaining Positive Pressure

Some embodiments of the invention relate to accessing the oral airway to perform procedures while maintain positive pressure performing oral care on a patient while maintaining sufficient pressure to provide clinically relevant breathing assistance and/or to maintain an open airway in the patient.

### (i) Oral Care

Described are methods for providing oral care through a non-invasive positive pressure ventilation mask. The method includes (i) providing a positive pressure ventilation mask including where the mask includes, a mask body defining a cavity and having a peripheral seal configured to engage a wearer's face, an access valve providing external access to the cavity, and an inlet for receiving pressurized air; (ii) pressurizing the mask to a pressure of at least 200 Pa using a ventilator system (alternatively to a pressure of at least 1, 2, 5, 10, or 15 and/or less than 35, 30, 25, 20, 15 cm H₂O, and/or within a range of any of the foregoing pressures); and (iii) introducing an oral appliance into the cavity through the valve and maintaining a pressure of at least 200 Pa (alternatively a pressure of at least 2, 5, 10, or 15 cm H₂O and/or less than 35, 30, or 25 cm H₂O) while performing an oral or nasal care procedure.

In a preferred embodiment, a bi-level ventilator is used and the two different pressures of the ventilator are different by at least 2, 4, 6, or 8 cm H₂0 and/or less than 20, 15, or 10 cm H₂0.

The desired pressure is maintained by providing an appliance with an adapter that couples to the mask and maintains sufficient seal, or by providing a valve in the mask that will seal around the appliance, or both. For example, in some embodiments, the self-sealing and/or self-reverting valve's described herein may be used for performing oral care. Alternatively, or in addition, an appliance adapter as described herein may be used with a port with a removable cap (see e.g., US Patent application publication 2010/0116276 to Bayasi). In either case, the valve opening or port is preferably positioned over the oral cavity and/or has a relatively wide opening (e.g., the dimensions described above) to facilitating oral cleaning, which requires a large degree of manipulation to be performed properly.

In some embodiments the oral care procedure includes applying a cleaning fluid to a least a portion of the oral cavity and suctioning excess cleaning fluid. The cleaning fluid may be water, mouth wash, or toothpaste. The cleaning fluid may include a cleaning agent such as a debriding agent or an anti-microbial agent. For example, the cleaning fluid may include a solution having an anti-microbial (e.g., cetylpyridinium chloride) in sufficient concentration to clean the oral cavity. For example, an antimicrobial may be added in a concentration of at least 0.01, 0.01, 0.05, or 0.1, or 0.5% and/or less than 1.0, 0.5, or 0.1% or within a range of the Anesthetics foregoing. For purposes of this invention, anesthetics suitable for numbing the throat (e.g., for performing intubation) are not a suitable "cleaning fluid" for performing the oral care described herein.

When cleaning the oral cavity, the cleaning fluid is typically applied to a majority of the surface of the mouth. In one embodiment, the cleaning fluid is applied to a least a portion of the gums and/or teeth.

The cleaning fluid may be provided in a container and a sponge on a stick may be dipped into the container to absorb an amount of solution. The sponge and fluid is then inserted into the oral cavity through a valve in the mask and the sponge is used to scrub surfaces of the mouth of the patient, include the teeth, gums, tongue, and/or cheeks of a patient.

In some embodiments, the fluid may be delivered using an appliance that also includes suction. The surface can be scrubbed using the sponge and fluid and fluid that is squeezed onto the surface can be suctioned through a shaft of the appliance. In one embodiment, the appliance can include a selectable suction controller such that the user performing the cleaning can select when to apply suction as the appliance is being used. For example, the appliance can have an access port to the suction channel and the port can be opened and close by the user covering it with his or her finger.

In some embodiments, the appliance may include bristles for more rigorous cleaning of surfaces (e.g., teeth).

The appliance inserted through the valve may have a shaft that is rigid and straight, which allows it to be moved in an appliance adapter while maintaining a seal. However, in some embodiments, the shaft may have a bend to allow easier access to the inside surface of the teeth.

The cleaning agent may be water or a mouth wash. include a toothpaste or other carrier. However, where a sponge appliance is desired, the cleaning agent is preferably a fluid.

Alternatively, or additionally, the method may include applying a wetting agent to the oral or nasal cavity. In some embodiments the wetting agent may include a moisturizer (e.g., methylcellulose). In some embodiments the wetting agent includes ice or ice water.

The method can include applying any anti-microbial or moisturizer known to be suitable for application to the mouth or nose for purposes of cleaning or wetting. Where a fluid is applied, the appliance used to deliver the fluid preferably includes suction. The use of an appliance to suction is important since the positive pressure is maintained during the treatment and it is important to avoid aspiration of excess fluids.

The method is a treatment of the nose, including treating epistaxis, lubricating the nasal cavity, and/or treating rhinorrhea. The appliance may also be used to treat itching or other irritations or discomforts. For example, a sponge on a stick can be used for mild scratching of the skin.

The ventilation system used to provide positive air pressure can be any known in the art, including volume ventilators, pressure controlled ventilators, bi-level positive airway pressure devices, continuous positive airway pressure devices, fixed pressure device, automatic positive airway pressure device, and expiratory positive airway pressure device.

The pressure during the oral or nasal care procedure is maintained at acceptable pressures for the patient such as a pressure of at least 300 Pa, 400 Pa, 500 Pa, or 600 Pa and/or less than 5000, 4000, 3000 Pa and/or within a range of the foregoing during use of an appliance in the valve.

To perform proper oral care, the valve has an opening of a suitable diameter. In one embodiment the valve has an opening with a diameter of at least 5, 10, 15, 20, or 25 mm and/or less than 60, 50, 40, 30, or 20, and/or within a range of the foregoing sizes. During use of the appliance in the mask the valve may be opened to a width within the foregoing ranges.

The method may be performed with a valve incorporated into the body of a mask. Alternatively, the valve may be incorporated into a conduit where the pressurized air flows into the inlet of the mask and the valve is configured to provide access to the cavity through the pressurized air inlet.

Unlike cleaning or wetting methods described in the prior art, the methods described can be carried out without removing the mask and without exceeding the maximum leak rate for the ventilator. This approach provides substantial benefits identified and recognized by the inventors. For example, by maintaining a suitable seal during use, the oral care adapter can be used on patients who are too ill to have the mask removed for oral care. In addition oral care may be performed by the patient themselves or a nurse, rather than a respiratory therapist (Although the RT or doctor can also perform the oral care). By facilitating a system that can be used by patients and nurses, the care providers can have sufficient resources to perform oral care on a regular basis, without substantially increasing the cost of care.

Described is an esophagogastroduodenoscopy (EGD) procedure under pressure. The method includes (i) providing a positive pressure ventilation mask including where the mask includes, a mask body defining a cavity and having a peripheral seal configured to engage a wearer's face, an access valve providing external access to the cavity, and an inlet for receiving pressurized air; (ii) pressurizing the mask to a pressure of at least 200 Pa using a ventilator system (alternatively to a pressure of at least 1, 2, 5, 10, or 15 and/or less than 35, 30, 25, 20, 15 cm H₂O, and/or within a range of any of the foregoing pressures); and (iii) introducing an endoscope into the cavity through the valve and maintaining a pressure of at least 200 Pa (alternatively a pressure of at least 2, 5, 10, or 15 cm H₂O and/or less than 35, 30, or 25 cm H₂O) while performing an EGD procedure. The endoscope may have suction and a working channel and/or a plurality of working channels and a diameter of at least 8 mm. The endoscope may include an appliance adapter configured to engage a port of the mask and seal the endoscope while allowing the endoscope to be advanced and/or manipulated in the patient's airway through the appliance adapter and the mask while maintaining the desire pressure.

The methods relate to performing an intubation through the masks and/or adapters described herein. The method can include advancing a bronchoscope through an airway that is at least partially maintained open using the positive pressure of the ventilator.

Described is to nebulize a patient using a nebulizer adapter that connects a nebulizer to the masks described herein. The nebulized gas can be introduced into the mask of the patient without being absorbed into the materials of the ventilation circuit and more immediately delivered in a more concentrated and accurately metered dosage. The nebulizer adapter may be connected and disconnected without disrupting the airflow to the patient.

Any of the forgoing methods may be carried out using an appliance adapter to deliver an appliance through the valve. The present invention can be incorporated into various masks and/or adapters using a variety of materials. Examples of positive air pressure masks that can be adapted to include a valve according to one embodiment of the invention are illustrated in U.S. Patent Application publications US2009/0194111 to Fu et al and US2010/0116276 to Bayasi. Methods are not limited to the novel masks and mask systems described herein. For example, the methods can be carried out using the mask of US 6,792,943 or 8,365,734 to Lehman. The foregoing patents and applications describe the teachings of masks and components that can be used in combination with the features of the present invention and their use for carrying out the methods described herein.

## Claims

1. A positive pressure ventilation (PPV) elbow (26) for accessing the oral cavity of a patient wearing a PPV mask under pressure, comprising:
a housing forming an elbow and defining an air-supply conduit extending between an air supply inlet (30) and an air supply outlet (34) the air supply conduit configured to deliver pressurized air from the inlet to the outlet;
a mask connector (32) configured to attach to a positive pressure ventilation mask; an access port formed through the housing into the conduit, the access port providing access to the mouth of a patient wearing the mask with the elbow connected thereto;
**characterized by** an access valve (42) positioned in the port (44) the access valve configured to self-seal under pressure from a ventilator.

2. The elbow connector as in claim 1, wherein the access valve self-seals at pressures between 5 and 15 cm H₂O.

3. The elbow connector as in any of the foregoing claims, wherein the access valve comprises a plurality of leaflets responsive to pressure for sealing the valve.

4. The elbow connector as in claim 3, wherein at least a portion of each leaflet has a layer of material at its center having a thickness of less than 2 mm.

5. The elbow connector as in any one of claims 3-4, wherein the access valve includes a locking rim (46) seated on the housing of the elbow and the leaflets are positioned within the conduit thereof.

6. The elbow connector as in any one of claims 3-5, wherein the leaflets, when inverted by an appliance, are self-reverting at pressures between 5 and 15 cm H₂O when the appliance is removed therefrom.

7. The elbow connector as in any one of claims 3-6, wherein the access valve includes one or more slits that allow the valve to be opened by an appliance, wherein the leaflets are configured to close the slit when pressure from the ventilator is applied to the leaflets and the appliance is removed from the valve.

8. The elbow connector as in any of the foregoing claims, wherein the access valve includes a cross slit.

9. The elbow connector as in any of the foregoing claims, wherein the access valve is a duckbill valve.

10. The elbow connector as in any of the foregoing claims, wherein the access valve opens to a diameter of at least 10 mm.

11. The elbow connector as in any of the foregoing claims, further comprising an anti-asphyxiation valve that opens to the ambient when the ventilator pressure is lost.

12. The elbow connector as in claim 1, wherein the mask connector is removably attaches to the mask.

13. A positive pressure ventilation mask comprising the elbow of claim 1.

14. A positive pressure ventilation mask as in claim 13, wherein the mask includes a mask shell and wherein the elbow swivels relative to the shell.

## Patentansprüche

1. Überdruck-Beatmungs-(PPV)-Winkelstück (26) zum Zugang zur Mundhöhle eines Patienten, der eine unter Druck stehende PPV-Maske trägt, umfassend:
ein Gehäuse, das ein Winkelstück bildet und eine Luftzufuhrleitung definiert, die sich zwischen einem Luftzufuhreinlass (80) und einem Luftzufuhrauslass (34) erstreckt, wobei die Luftzufuhrleitung konfiguriert ist, um Druckluft vom Einlass zum Auslass zu liefern;
einen Maskenverbinder (32), der konfiguriert ist, um an einer Überdruck-Beatmungsmaske befestigt zu werden;
eine Zugangsöffnung, die durch das Gehäuse in die Leitung gebildet ist, wobei die Zugangsöffnung Zugang zum Mund eines Patienten bereitstellt, der die Maske trägt, wobei der Winkelstück damit verbunden ist;
**gekennzeichnet durch** ein Zugangsventil (42), das in der Öffnung (44) positioniert ist, wobei das Zugangsventil konfiguriert ist, um sich unter Druck von einem Beatmungsgerät selbst abzudichten.

2. Winkelverbinder nach Anspruch 1, wobei das Zugangsventil bei Drücken zwischen 5 und 15 cm H₂O selbstabdichtend ist.

3. Winkelverbinder nach einem der vorhergehenden Ansprüche, wobei das Zugangsventil eine Vielzahl von Blättchen umfasst, die auf Druck ansprechen, um das Ventil abzudichten.

4. Winkelverbinder nach Anspruch 3, wobei mindestens ein Abschnitt jedes Blättchens in seiner Mitte eine Materialschicht mit einer Dicke von weniger als 2 mm aufweist.

5. Winkelverbinder nach einem der Ansprüche 3 bis 4, wobei das Zugangsventil einen Verschlussrand (46) umfasst, der auf dem Gehäuse des Winkelstücks sitzt, und die Blättchen in dessen Leitung positioniert sind.

6. Winkelverbinder nach einem der Ansprüche 3 bis 5, wobei die Blättchen, wenn sie von einem Gerät umgedreht werden, bei Drücken zwischen 5 und 15 cm H₂O selbst umkehren, wenn das Gerät davon entfernt wird.

7. Winkelverbinder nach einem der Ansprüche 3 bis 6, wobei das Zugangsventil einen oder mehrere Schlitze beinhaltet, die es ermöglichen, dass das Ventil durch ein Gerät geöffnet wird, wobei die Blättchen konfiguriert sind, um den Schlitz zu schließen, wenn Druck von dem Beatmungsgerät auf die Blättchen ausgeübt wird und das Gerät von der Klappe entfernt wird.

8. Winkelverbinder nach einem der vorhergehenden Ansprüche, wobei das Zugangsventil einen Querschlitz umfasst.

9. Winkelverbinder nach einem der vorhergehenden Ansprüche, wobei das Zugangsventil ein Entenschnabelventil ist.

10. Winkelverbinder nach einem der vorhergehenden Ansprüche, wobei sich das Zugangsventil bis zu einem Durchmesser von mindestens 10 mm öffnet.

11. Winkelverbinder nach einem der vorhergehenden Ansprüche, ferner umfassend ein Anti-Erstickungsventil, das sich zur Umgebung öffnet, wenn der Beatmungsdruck verloren geht.

12. Winkelverbinder nach Anspruch 1, wobei der Maskenverbinder abnehmbar an der Maske befestigt ist.

13. Überdruck-Beatmungsmaske, umfassend das Winkelstück nach Anspruch 1.

14. Überdruck-Beatmungsmaske nach Anspruch 13, wobei die Maske eine Maskenschale umfasst, und wobei das Winkelstück relativ zur Schale schwenkt.

## Revendications

1. Coude (26) de ventilation en pression positive (PPV) pour accéder à la cavité buccale d'un patient portant un masque PPV sous pression, comprenant :
un boîtier formant un coude et définissant une conduite d'alimentation en air s'étendant entre une entrée d'alimentation en air (30) et une sortie d'alimentation en air (34), la conduite d'alimentation en air étant configurée pour fournir de l'air sous pression de l'entrée à la sortie ;
un connecteur de masque (32) configuré pour se fixer à un masque de ventilation en pression positive ;
un orifice d'accès formé à travers le boîtier dans la conduite, l'orifice d'accès fournissant un accès à la bouche d'un patient portant le masque avec le coude connecté à celui-ci ;
**caractérisé par** un clapet d'accès (42) positionné dans l'orifice (44), le clapet d'accès étant configuré pour se sceller automatiquement sous pression à partir d'un ventilateur.

2. Connecteur coudé selon la revendication 1, dans lequel le clapet d'accès se scelle automatiquement à des pressions comprises entre 5 et 15 cm H₂O.

3. Connecteur coudé selon l'une quelconque des revendications précédentes, dans lequel le clapet d'accès comprend une pluralité de feuillets sensibles à la pression pour fixer de manière étanche le clapet.

4. Connecteur coudé selon la revendication 3, dans lequel au moins une partie de chaque feuillet a une couche de matière en son centre ayant une épaisseur inférieure à 2 mm.

5. Connecteur coudé selon l'une quelconque des revendications 3 à 4, dans lequel le clapet d'accès comporte un anneau de verrouillage (46) installé sur le boîtier du coude et les feuillets sont positionnés à l'intérieur de la conduite de celui-ci.

6. Connecteur coudé selon l'une quelconque des revendications 3 à 5, dans lequel les feuillets, lorsqu'ils sont inversés par un appareil, sont autoréversibles à des pressions comprises entre 5 et 15 cm H₂O lorsque l'appareil est retiré de ceux-ci.

7. Connecteur coudé selon l'une quelconque des revendications 3 à 6, dans lequel le clapet d'accès comporte une ou plusieurs fentes qui permettent au clapet d'être ouvert par un appareil, dans lequel les feuillets sont configurés pour fermer la fente lorsque la pression du ventilateur est appliquée aux feuillets et que l'appareil est retiré du clapet.

8. Connecteur coudé selon l'une quelconque des revendications précédentes, dans lequel le clapet d'accès comporte une fente transversale.

9. Connecteur coudé selon l'une quelconque des revendications précédentes, dans lequel le clapet d'accès est un clapet en bec de canard.

10. Connecteur coudé selon l'une quelconque des revendications précédentes, dans lequel le clapet d'accès s'ouvre sur un diamètre d'au moins 10 mm.

11. Connecteur coudé selon l'une quelconque des revendications précédentes, comprenant en outre un clapet anti-asphyxie qui s'ouvre à l'air ambiant lorsque la pression du ventilateur est perdue.

12. Connecteur coudé selon la revendication 1, dans lequel le connecteur de masque est fixé de manière amovible au masque.

13. Masque de ventilation en pression positive comprenant le coude selon la revendication 1.

14. Masque de ventilation en pression positive selon la revendication 13, dans lequel le masque comporte une coque de masque et dans lequel le coude pivote par rapport à la coque.
